# EUROPEAN PATENT APPLICATION

(11) **EP 3 692 997 A1**
(43) Date of publication of application: **12.08.2020**
(21) Application number: 18864902.4
(22) Date of filing: 10.08.2018
(51) Int. Cl.: A61K 35/17, A61K 35/76, A61K 39/145, A61K 39/275, A61K 39/395, A61K 45/00, A61P 31/16

(54) **MEDICATION FOR INFLUENZA**

(30) Priority: 03.10.2017 JP 2017193452
(71) Applicant: Tokyo Metropolitan Institute of Medical Science, Tokyo 156-8506 (JP)
(72) Inventor: YASUI Fumihiko, Tokyo 156-8506 (JP); KOHARA Michinori, Tokyo 156-8506 (JP)
(74) Representative: J A Kemp LLP
(86) International application number: PCT/JP2018/030141
(87) International publication number: WO 2019/069561

(57) **Abstract**

A medication for preventing or treating influenza, said medication including an inactivated influenza virus or a recombinant vaccinia virus that includes, within the genome of the vaccinia virus DIs strain, an expression promoter and all or a portion of DNA coding for an influenza virus-derived protein.

## Description

### FIELD OF THE INVENTION

The present invention relates to a medicine for preventing and treating onset of influenza resulting from influenza virus infection.

### BACKGROUND ART

Seasonal influenza epidemics occur every winter, where 10 to 20% of the population is reported to be affected. The pathogen of such influenza could be influenza virus type A/HlN1, A/H3N2 or B but their antigenicity slightly changes every year and thus a vaccine needs to be produced in accordance with the strain that is predicted to dominate. Meanwhile, there are new types of influenza such as highly pathogenic H5N1 avian influenza and H7N9 avian influenza. Specifically, a patient infected with highly pathogenic H5N1 avian influenza virus (H5N1 HPAIV) was first reported in 1997, where the number of infected persons expanded since 2003 mainly among the regions in China, Southeast Asia and the Middle East. To date, 800 or more infected cases and 450 or more deaths, that is, about 50% of the cases, have been reported. Currently, pre-pandemic vaccines produced by forecasting the epidemic strains for four clades (clades 1, 2.1, 2.2 and 2.3) are stockpiled as H5N1 HPAIV vaccines in forms of inactivated whole virus particle vaccines against viruses that have been propagated by inoculating embryonated chicken eggs.

Since, however, inoculation of embryonated chicken eggs with H5N1 HPAIV is highly lethal and thus the virus yield is low, there is a problem of having difficulty in mass preparation. Actually, the pre-pandemic H5N1 HPAIV vaccines manufactured and stockpiled in Japan have been subjected to three clinical tests, namely, "safety test" that was conducted with 6000 participants from 2008 to 2009, "primary vaccination test" targeting 200 participants, and "booster vaccination test" targeting 200 participants who had already received the primary vaccination. Their safety was stated to be within the expected range. At the same time, while cross-reactivity was confirmed in the "booster vaccination test" where subjects who had already received primary vaccination with H5N1 "Vietnam strain" vaccine were immunized with a different vaccine strain, i.e., Indonesia strain or Anhui strain, induction of neutralizing antibodies against different virus strains was reported to be insufficient in the "primary vaccination test" in which the subjects were immunized with the same vaccine twice with a 3-week interval. Moreover, cross-reactivity between the subtypes, especially between clades 2.2 and 2.3 among these four types of vaccines prepared by a similar technique on a laboratory scale, was reported to be low and thus lethality was not fully suppressed in an experiment of protection against infection using mouse models (Non-patent document 1). After all, since these vaccines were prepared by predicting the epidemic strains, there are many questionable points as to whether or not they can exert vaccine effectiveness against the actually emerging epidemic strain.

At present, therapeutic agents for influenza include Tamiflu and Relenza that inhibit the function of neuraminidase (NA) that is essential for the budding of the influenza virus, but their effectiveness is limited because, for example, it is critical to administer them within 48 hours following the onset. Beside them, an intravenous agent of a NA inhibitor has already been developed. Furthermore, although manufacturing and sales of Avigan, an influenza virus polymerase inhibitor, were approved in Japan in March 2014, it can only be manufactured and distributed under certain conditions such as upon a request from the Minister of Health, Labour and Welfare and cannot be sold in the general market.

Considering this situation, establishment of a prophylactic and therapeutic medicine that is widely and extendedly effective against an influenza virus, whose epidemic is hard to forecast, has been strongly needed.

Among the variety of vaccines, live vaccines are one of the most effective vaccines, but development of an attenuated vaccine for an emerging virus is generally known to take a very long time. So far, recombinant vaccinia viruses (RVV) against rabies virus and rinderpest developed by the present inventors have been demonstrated to exert an excellent effect in preventing infection and onset in the field tests and else (Non-patent document 2). In addition, for severe acute respiratory syndrome (SARS), production of a recombinant vaccinia virus that has cDNA of SARS-CoV known as a pathogen thereof has also been successful, and it has been confirmed to be a formulation that shows an excellent preventive effect and that can be repeatedly administered (Non-patent document 3). Furthermore, for H5N1 HPAIV, production of a recombinant vaccinia virus having cDNA of H5N1 HPAIV HA has also been successful, and it has been confirmed to be a formulation that shows an excellent preventive effect (Non-patent document 4).

### PRIOR ART DOCUMENTS

### Patent document

Patent document 1: International Patent Application Publication No. 2006/038742

### Non-patent documents

Non-patent document 1: Muarakami S. et al., Cross-clade protective immunity of H5N1 influenza vaccines in a mouse model., Vaccine, vol. 26(50), p. 6398-6404,2008.
Non-patent document 2: Tsukiyama K. et al., Development of heat-stable recombinant rinderpest vaccine. Arch. Virol., 1989, vol. 107, p. 225-235
Non-patent document 3: Kitabatake M. et al., SARS-CoV spike protein-expressing recombinant vaccinia virus efficiently induces neutralizing antibodies in rabbits pre-immunized with vaccinia virus. Vaccine, 2007, vol. 25, p. 630-637
Non-patent document 4: Yasui F. et al., Sensitization with vaccinia virus encoding H5N1 hemagglutinin restore immune potential against H5N1 influenza virus. Sci. Rep., 2016, vol. 6, p. 37915

### SUMMARY OF INVENTION

### Problem to be Solved by the Invention

The problem to be solved by the present invention is to provide an anti-influenza drug that is effective in inhibiting onset resulting from infection caused by influenza viruses including H5N1 HPAIV.

### Means for Solving Problem

The present inventors have gone through further intensive investigation based on the results of the past analyses and evaluations in relation to infection with new types of influenza viruses, and considered that use of a recombinant vaccinia vaccine for inducing strong immune activation can lead to a potent method for preventing influenza virus infection. In addition, they also found that a vaccinia virus DIs strain can be used as a virus strain for producing a recombinant vaccinia virus so as to solve the above-described problem, for example, by protecting against the onset caused by challenge infection with strains of different clades by a single immunization. At the same time, they found that the above-described problem can be solved, unlike the conventional inactivated vaccines that depend on the neutralizing antibodies, since its protective action is exerted via a mechanism independent from the neutralizing antibody titer, thereby accomplishing the present invention.

Thus, the present invention is as follows.
(1) A medication for preventing or treating influenza, comprising a recombinant vaccinia virus that contains an expression promoter and the entire or a part of DNA coding for an influenza virus-derived protein within a genome of a vaccinia virus DIs strain, or an inactivated influenza virus.
(2) The medication according to (1), wherein influenza is prevented or treated by the action of an activated CD4-positive cell and/or an activated CD8-positive cell.
(3) The medication according to either one of (1) and (2), further comprising an antibody against an influenza virus-derived protein.
(4) The medication according to (3), wherein the antibody is a binding antibody.
(5) The medication according to any one of (1)-(4), wherein the influenza virus-derived protein is a hemagglutinin protein derived from highly pathogenic H5N1 avian influenza virus.
(6) A medication for preventing or treating influenza, comprising any one of components (a)-(c) below:
   (a) at least one component selected from the group consisting of an activated CD4-positive cell, an activator of a CD4-positive cell and a combination of a CD4-positive cell and an activator of a CD4-positive cell;
   (b) at least one component selected from the group consisting of an activated CD8-positive cell, an activator of a CD8-positive cell and a combination of a CD8-positive cell and an activator of a CD8-positive cell; and
   (c) a component consisting of a combination of the components (a) and (b).
(7) The medication according to (6), further comprising a recombinant vaccinia virus that contains an expression promoter and the entire or a part of DNA coding for an influenza virus-derived protein within a genome of a vaccinia virus DIs strain, or an inactivated influenza virus.
(8) The medication according to either one of (6) and (7), further comprising an antibody against an influenza virus-derived protein.
(9) The medication according to (8), wherein the antibody is a binding antibody.
(10) The medication according to any one of (7)-(9), wherein the influenza virus-derived protein is a hemagglutinin protein derived from highly pathogenic H5N1 avian influenza virus.
(11) A kit for preventing or treating influenza, comprising a recombinant vaccinia virus that contains an expression promoter and the entire or a part of DNA coding for an influenza virus-derived protein within a genome of a vaccinia virus DIs strain, or an inactivated influenza virus.
(12) The kit according to (11), wherein influenza is prevented or treated by the action of an activated CD4-positive cell and/or an activated CD8-positive cell.
(13) The kit according to either one of (11) and (12), further comprising an antibody against an influenza virus-derived protein.
(14) The kit according to (13), wherein the antibody is a binding antibody.
(15) The kit according to any one of (11)-(14), wherein the influenza virus-derived protein is a hemagglutinin protein derived from highly pathogenic H5N1 avian influenza virus.
(16) A kit for preventing or treating influenza, comprising any one of components (a)-(c) below:
   (a) at least one component selected from the group consisting of an activated CD4-positive cell, an activator of a CD4-positive cell and a combination of a CD4-positive cell and an activator of a CD4-positive cell;
   (b) at least one component selected from the group consisting of an activated CD8-positive cell, an activator of a CD8-positive cell and a combination of a CD8-positive cell and an activator of a CD8-positive cell; and
   (c) a component consisting of a combination of the components (a) and (b).
(17) The kit according to (16), further comprising a recombinant vaccinia virus that contains an expression promoter and the entire or a part of DNA coding for an influenza virus-derived protein within a genome of a vaccinia virus DIs strain, or an inactivated influenza virus.
(18) The kit according to either one of (16) and (17), further comprising an antibody against an influenza virus-derived protein.
(19) The kit according to (18), wherein the antibody is a binding antibody.
(20) The kit according to any one of (17)-(19), wherein the influenza virus-derived protein is a hemagglutinin protein derived from highly pathogenic H5N1 avian influenza virus.

### EFFECT OF THE INVENTION

The present invention can provide a novel medicine for preventing or treating influenza (a prophylactic or therapeutic vaccine for influenza) which is effective in preventing or treating influenza and which is highly safe.

### BRIEF DESCRIPTION OF THE DRAWINGS

[Figure 1] Diagrams showing a gene structure of a plasmid vector for producing a recombinant vaccinia virus (RVV) having a HA gene of a new type of influenza virus (H5N1 HPAIV) (specifically, rDIs/mC11, rDIs/mC12.1, rDIs/mC12.2 or rDIs/mC12.3). In the figure, "Influenza HA" refers to the HA gene (cDNA) of each influenza virus while "Subtype H5" refers to H5N1 HPAIV (the same also applies to other figures).
[Figure 2] Diagrams showing the results of the evaluations of the protective effect against H5N1 HPAIV challenge infection in BALB/c mice by a single immunization with a DIs strain-derived RVV having a H5N1 HPAIV-derived HA gene (H5N1 HPAIV vaccine: rDIs/mC12.2).
[Figure 3] A diagram showing viral loads in lung tissues from the day of the H5N1 HPAIV challenge infection to the following 9 days, in BALB/c mice that were given a single immunization with a H5N1 HPAIV vaccine (rDIs/mC12.2).
[Figure 4] A diagram showing neutralizing titers from the day of the H5N1 HPAIV challenge infection to the following 6 days, in BALB/c mice that were given a single immunization with a H5N1 HPAIV vaccine (rDIs/mC12.2).
[Figure 5] Diagrams showing viral loads from the day of the virus infection to the following 6 days, in BALB/c mice given a binding antibody against the HA protein.
[Figure 6] Diagrams showing changes in the weight from the day of the H5N1 HPAIV challenge infection to the following 9 days and viral loads in lung tissues 1 and 3 days following the virus infection, in wild-type mice and Fc receptor gamma knockout mice immunized with a H5N1 HPAIV vaccine (rDIs/mC12.2).
[Figure 7] A diagram showing the influence of CD4-positive cell depletion on the viral loads in lung tissues from the day of the H5N1 HPAIV challenge infection to the following 6 days, in BALB/c mice that were given a single immunization with a H5N1 HPAIV vaccine (rDIs/mC12.2).
[Figure 8] A diagram showing the influence of CD8-positive cell depletion or depletion of both CD4- and CD8-positive cells on the viral loads in lung tissues of the vaccinated group.
[Figure 9] Diagrams showing the influence of depletion of both CD4- and CD8-positive cells on the weight and the survival rate of the vaccinated group.

### MODE FOR CARRYING OUT THE INVENTION

Hereinafter, the present invention will be described in detail.

The scope of the present invention should not be limited to the following description, and the present invention may suitably be modified and carried out in any way apart from the following illustration without departing from the gist of the present invention. The patent documents, the non-patent documents and other publications cited herein are incorporated herein by reference.

### 1. Outline of the present invention

The current pre-pandemic vaccines against H5N1 HPAIV are whole particle vaccines obtained by generating an influenza virus that has the multibasic site of H5N1 HA deleted by a reverse genetic technique, and inactivating the resultant with formalin. However, it requires immunizations twice with a 3-week interval in order to induce an effective immune response, and its preventive effect is significantly weakened against a virus strain of a clade different from that of the vaccine used for immunization.

Meanwhile, a genetic engineering technique for preparing a recombinant vaccinia virus (RVV) as a live vaccine is known. A vaccinia virus that serves as a recombinant parent for preparing a RVV needs to be a vaccine strain with established safety, where such vaccine strains include vaccinia virus DIs strain and LC16m8 strain. The vaccinia virus DIs strain is a highly attenuated vaccinia virus strain separated by passaging vaccinia virus Dairen strain (DEI strain) in chicken egg embryos (Tagaya I, et al., A new mutant of dermovaccinia virus. Nature, 1961, vol. 192, p. 1187-1188), which can propagate in chicken embryonic fibroblasts (CEF) but is non-proliferative in other mammal cells and thus is highly safe. Meanwhile, vaccinia virus LC16m8 strain was approved as a smallpox vaccine by the then Ministry of Health and Welfare in 1975, and is a safe and effective attenuated vaccine strain that has actually been used for immunizing more than 50,000 infants. Attenuation of the LC16m8 strain owes to the fact that the full-length B5R protein is not expressed due to a single-nucleotide deletion in the B5R protein gene of LC16mO strain that is considered to be responsible for adhesion to cells and infection (Morikawa S., et al. An attenuated LC16m8 smallpox vaccine: analysis of full-genome sequence and induction of immune protection. J. Virol., 2005, vol. 79(18), p. 11873-1189).

Accordingly, a recombinant live influenza vaccine whose safety is highly likely ensured even when used for immunizing a person undergoing immunodeficiency or immunosuppression, and which has advantages such as leaving no vaccine scar can be developed. The present inventors established a new type of recombinant influenza vaccine by using this DIs strain as a parent, which showed effectiveness against H5N1 HPAIV infection by a single immunization. Specifically, as a H5N1 HPAIV vaccine (rDIs/mC12.2), a DIs strain-derived RVV expressing an influenza virus HA gene shows rapid weight gaining and 100% survival rate even against lethal H5N1 HAPIV challenge infection, by a single immunization with 1 x 10⁷ PFU of the RVV. Thus, the DIs strain-derived RVV is sufficiently effective as a H5N1 HPAIV vaccine.

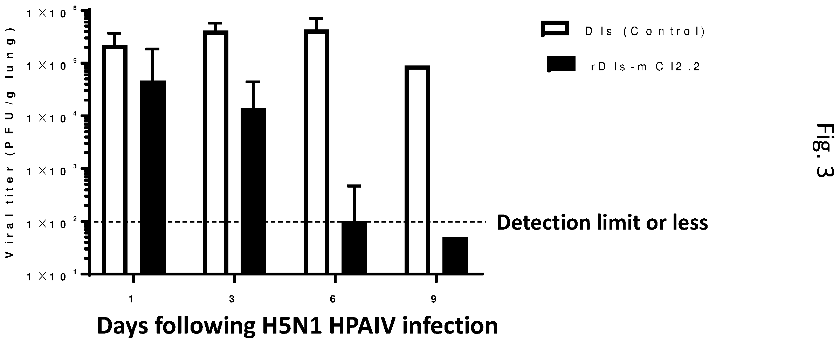

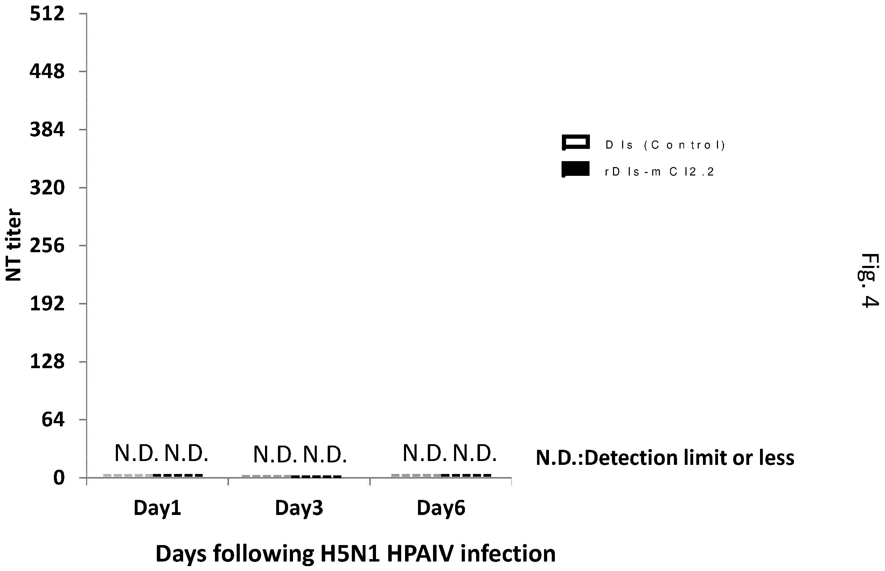

Besides, a satisfying preventive effect was observed in individuals immunized with the H5N1 HPAIV vaccine (rDIs/mC12.2) even without the induction of the neutralizing antibody against H5N1 HPAIV (Figures 3 and 4).

Thus, the present invention aimed at elucidating a protective effect that does not depend on the neutralizing antibody.

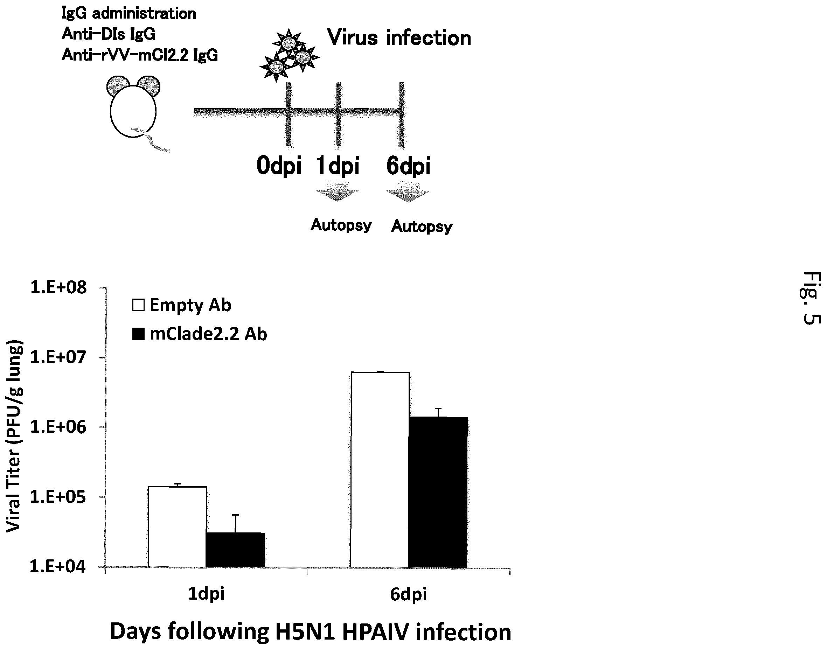

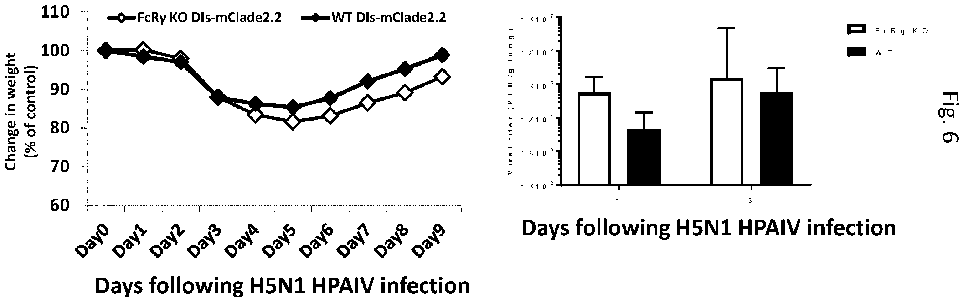

As a result, in individuals immunized with a H5N1 HPAIV vaccine (rDIs/mCl2.2), namely, a vaccinia virus DIs strain incorporating a gene coding for a hemagglutinin (HA) protein of highly pathogenic H5N1 avian influenza virus (H5N1 HPAIV) and an expression promoter, various cell groups and factors were found to be involved in the protection against the infection with days following the infection. Specifically, the virus was generally eliminated to the detection limit or less by 6 days after the H5N1 HPAIV infection in the individuals immunized with the H5N1 HPAIV vaccine (rDIs/mC12.2). In these vaccinated individuals, the binding antibody produced after the vaccination contributed to the protection against virus infection probably via FcRg-mediated ADCC where the binding antibody and a cell group having a Fc gamma receptor contributed to the infection protection until a day after the H5N1 HPAIV infection. The contribution of the antibody to the virus elimination, however, was shown to be not as high as to result the virus elimination observed in the individuals immunized with the H5N1 HPAIV vaccine (rDIs/mC12.2) (Figure 3) (Figures 5 and 6).

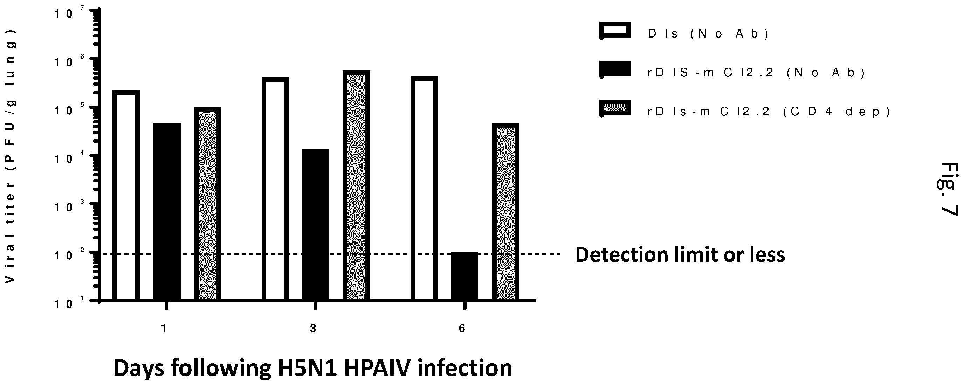

On the other hand, the results of a CD4-positive cell depletion experiment showed that the CD4-positive cell was essential for the protection against the virus infection from Day 1 to 3 following the infection in the individuals immunized with the H5N1 HPAIV vaccine (rDIs/mC12.2) (Figure 7).

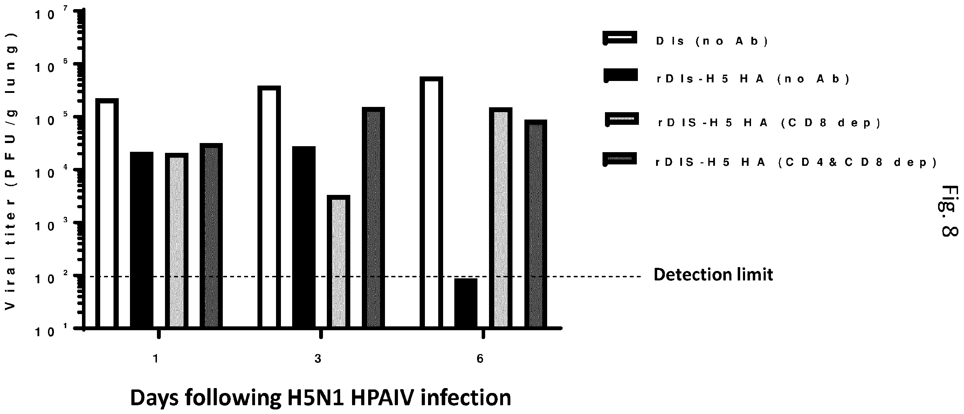

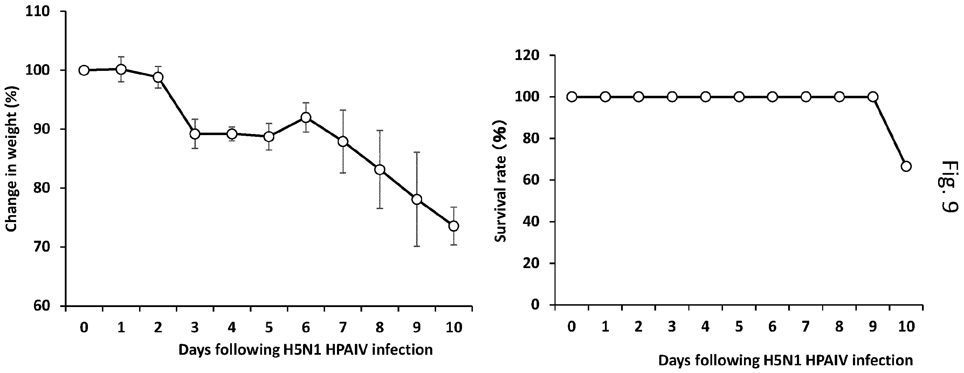

Furthermore, the CD8-positive cell was found to play an important role in the virus elimination from Day 3 to 6 following the infection (Figure 8). Moreover, when both of the CD4- and CD8-positive cells were depleted, virus was not eliminated in the vaccinated individuals even 10 days after the infection, and the individuals significantly lost weight and began to die (Figure 9).

These results show that, unlike an antigen-specific antibody (in particular, neutralizing antibody) that has been considered important for the preventive action of the conventional vaccination with an inactivated whole particle, the HPAIV vaccine (rDIs/mC12.2) exerts a protective effect against H5N1 HPAIV infection by the mechanism of strong anti-influenza viral action mediated by the CD4- and/or CD8-positive cells.

The aforementioned results show that a recombinant vaccinia virus that contains an expression promoter and the entire or a part of DNA coding for an influenza virus-derived protein within a genome of a vaccinia virus DIs strain, or an inactivated influenza virus can be used as a medication for preventing or treating influenza or as a vaccine for preventing or treating influenza. According to the present invention, the effect of preventing or treating influenza is exerted via the action of an activated CD4-positive cell, an activated CD8-positive cell or a combination thereof.

In addition, an activated CD4-positive cell and/or an activated CD8-positive cell against an influenza virus HA protein was found to serve as a safe and effective prophylactic or therapeutic medicine against a rapidly spreading influenza virus.

### 2. Activator of CD4/CD8-positive cells and activated CD4/CD8-positive cells

In one aspect of the present invention, an activated CD4-positive cell, an activator of a CD4-positive cell or a combination of an activator of a CD4-positive cell and a CD4-positive cell is used as a medication (prophylactic or therapeutic pharmaceutical composition) for preventing or treating influenza.

In one aspect of the present invention, an activated CD8-positive cell, an activator of a CD8-positive cell or a combination of a CD8-positive cell and an activator of a CD8-positive cell is used as a medication (prophylactic and therapeutic pharmaceutical composition) for preventing or treating influenza.

Hereinafter, a CD4-positive cell, a CD8-positive cell or both of CD4- and CD8-positive cells are also referred to as "CD4/CD8-positive cells".

Moreover, in one aspect of the present invention, DIs strain-derived RVV-H5N1 HPAIV (rDIs/mC12.2) or the like or an inactivated influenza virus can be used as an inactivated vaccine together with the aforementioned activated CD4/CD8-positive cells, the aforementioned activator of the CD4/CD8-positive cells or the aforementioned combination of the CD4/CD8-positive cells and the activator of the CD4/CD8-positive cells.

CD4-positive cells may be helper T cells (which can be classified into Th1, Th2 or Th17) or regulatory T cells. The T cell receptor (TCR) of the CD4-positive cell binds to a macrophage and a Class II MHC molecule of a B cell. The helper T cell recognizes an antigen presented by a macrophage or the like via the Class II MHC molecule and activates the B cell and a cytotoxic T cell. Meanwhile, the regulatory T cell is involved in shutting down the immune responses.

CD8 cells are T cells that are responsive to an antigen presented on a Class I MHC molecule, and that act as cytotoxic T cells that identify said antigen and destroy the virus-infected cells. In addition, they affect the virus-infected cells via production of interferon gamma (IFN-g) and break down the intracellular antigen in a non-cytotoxic way.

The inactivated influenza virus refers to one that is obtained by propagating an influenza virus used for vaccine production by inoculating embryonated chicken eggs with said influenza virus, then partially degrading the virus purified/concentrated from the chorioallantoic fluid with ether, and further inactivating the resultant with formalin. According to the present invention, however, one that is obtained by inactivating the virus particle itself can also be used.

The CD4/CD8-positive cells can be collected from blood, bone marrow or the like of a subject and can be acquired using a cell sorter or the like. Alternatively, it may be derived from a stem cell such as iPS.

Whether or not the collected cells are CD4/CD8-positive cells can be confirmed by flow cytometry.

Examples of the activator of the CD4-positive cell include interleukin (IL)-2, IL-4, CD86, OX40 ligand, a HA protein as the antigen and a part thereof. The CD4-positive cell is activated by culturing it with the aforementioned activator.

Examples of the activator of the CD8-positive cells include IL-2, IL-12, IFN-g, CD86, CD137 ligand, OX40 ligand, a HA protein as the antigen and a part thereof. The CD8-positive cell is activated by culturing with the aforementioned activator.

Whether or not the CD4/CD8-positive cells have been activated can be confirmed by verifying cytokine production by flow cytometry or ELISpot or by an increase in the expression of the activation marker.

At the same time, according to the present invention, when a CD4/CD8-positive cell activator is administered to a subject (healthy person or patient), CD4/CD8-positive cells originating in the subject are activated in the body of the subject. The mechanism of the activation of the CD4/CD8-positive cells in a body is as follows: when a CD4-positive cell activator such as IL-2, IL-4, CD86, OX40 ligand, a HA protein as the antigen or a part thereof is administered, expression of surface molecules such as CD40L, CD44 and CD69 is increased, which results in activation of the CD4-positive cells. In addition, when a CD8-positive cell activator such as IL-2, IL-12, IFN-g, CD86, CD137 ligand, OX40 ligand, a HA protein as the antigen or a part thereof is administered, expression of surface molecules such as CD44 and CD69 is increased, which results in activation of the CD8-positive cells.

Furthermore, a combination of CD4/CD8-positive cells with an activator of CD4/CD8-positive cells can be administered to a subject so that the administered CD4/CD8-positive cells are activated together with the CD4/CD8-positive cells inherent in the body of the subject. Whether or not these CD4/CD8-positive cells are activated can be confirmed by verifying cytokine production by flow cytometry or ELISpot or by an increase in the expression of the activation marker.

### 3. Antibody against influenza virus-derived protein

In another aspect of the present invention, an antibody against an influenza virus-derived protein is used together with the recombinant vaccinia virus or the inactivated influenza virus of the present invention, as a medication for preventing or treating influenza.

Moreover, in a further aspect of the present invention, an antibody against an influenza virus-derived protein is used together with an activator of CD4/CD8-positive cells and/or activated CD4/CD8-positive cells, as a medication for preventing or treating influenza.

Examples of the influenza virus-derived protein used for the present invention include, but not limited to, influenza H1N1, H3N2 and B viruses. According to the present invention, a H5N1 HPAIV-derived hemagglutinin (HA) protein is mainly used.

Alternatively, the aforementioned antibody against an influenza virus-derived protein or an antigen-binding fragment thereof can be used as a medication for preventing or treating influenza.

The antibody may be either a binding antibody against an influenza virus-derived protein or a neutralizing antibody against an influenza virus-derived protein. These antibodies may be polyclonal or monoclonal antibodies, or a fragment thereof. If a H5N1 HPAIV-derived HA protein is to be used as an antigen, an antibody against said protein (which is referred to as an "anti-HA antibody") can recognize the HA protein existing in an environment of a living body (HA protein having an intact structure).

A "binding antibody" refers to an antibody that recognizes an influenza virus-derived protein (for example, a HA protein of H5N1 virus) as an antigen, and has a function of opsonization or a function similar to opsonization. While a binding antibody has a function of binding to an antigen, it does not have a neutralizing activity against an influenza virus (for example, H5N1 HPAIV).

A "neutralizing antibody" refers to an antibody that recognizes an influenza virus-derived protein (for example, a HA protein of H5N1 virus) as an antigen, and that is produced from an individual who has received the later-described DIs vaccination. A neutralizing activity of a neutralizing antibody refers to a function of inhibiting adhesion and/or infection of a virus to a target cell. Specifically, the function of a neutralizing antibody refers to a function of inhibiting adhesion of a protein of the virus to a receptor on the surface of a target cell and infection of the cell, by binding to the influenza virus-derived protein regardless of where it binds.

The antibody of the present invention has an activity of binding to polypeptides having the amino acid sequences represented by the accession numbers indicated in Table 1 below. Apart from that, it may recognize a mutant polypeptide having substitution, deletion or insertion of one or several (for example, 2, 3, 4 or 5) amino acids in said amino acid sequence, or a mutant polypeptide having 80% or more, preferably 90% or more, 95% or more, 98% or more, or 99% or more identity to the amino acid sequence represented by said sequence number.

In a further aspect of the present invention, the antibody of the present invention comprises an antibody that recognizes a polypeptide coded by a polynucleotide consisting of the nucleotide sequence represented by the accession number shown in Table 1, or a mutant polypeptide coded by a polynucleotide having 80% or more, 90% or more, 95% or more, 98% or more, or 99% or more identity to said nucleotide sequence.

In a further aspect of the present invention, the antibody of the present invention may be an antibody that recognizes a mutant polypeptide that is coded by a polynucleotide containing a nucleotide sequence that hybridizes with the nucleotide sequence represented by the accession number shown in Table 1 under highly stringent conditions.

### <Neutralizing antibody>

As mentioned above, the neutralizing antibody of the present invention is an antibody produced from an individual who has received a DIs vaccination.

An immunization technique of administering a DIs vaccine alone or together with a carrier and a diluent to a non-human mammal, for example, a rabbit, a dog, a guinea pig, a mouse, a rat, a goat, a horse, a sheep or the like for immunization is well known.

A neutralizing titer of an antibody in a serum can be determined by a micro-neutralization assay or the like. After confirming that the neutralizing titer has increased sufficiently, whole blood is collected to separate/purify the antibody by a generally employed method. Separation/purification can be carried out by appropriately selecting or combining known methods such as ammonium sulfate precipitation, ion exchange chromatography, gel filtration chromatography and affinity chromatography.

### <Binding antibody>

Since the binding antibody of the present invention is an antibody against an influenza virus-derived protein (for example, a HA protein of a H5N1 virus), said protein or a partial peptide thereof can be used as an antigen to produce the antibody in the same manner as a method for producing a general polyclonal or monoclonal antibody.

### 4. Production of antibody

### (1) Production of polyclonal antibody

An influenza virus-derived protein or a partial peptide is administered alone or together with a carrier and a diluent to a non-human mammal, for example, a rabbit, a dog, a guinea pig, a mouse, a rat, a goat or the like for immunization. The dosage of the antigen per animal, the dosage when an adjuvant is used, the type of the adjuvant, the immunized site, the interval between the doses and the like are well known.

Determination of an antibody titer in a serum is also well known, and may be carried out by ELISA, EIA, RIA or the like. After confirming that the antibody titer has increased sufficiently, whole blood is collected to separate/purify the antibody by a generally employed method. Separation/purification can be carried out by appropriately selecting or combining known methods such as ammonium sulfate precipitation, ion exchange chromatography, gel filtration chromatography and affinity chromatography.

### (2) Preparation of monoclonal antibody

A method for preparing a monoclonal antibody is also well known. For example, harvesting of antibody-producing cells, cell fusion between the antibody-producing cells and myeloma cells for obtaining hybridomas, selection and cloning of the hybridoma, harvesting of the monoclonal antibody and the like can be carried out according to methods well known to those skilled in the art.

Furthermore, according to the present invention, an epitope (antigenic determinant) of the antibody is not limited as long as it is at least a part of the influenza virus-derived protein, i.e., the antigen. The antibody of the present invention comprises an antibody that binds to a site (for example, an epitope) where said antibody binds, for example, an antibody that binds to a site where an antibody produced by the hybridoma of the present invention binds.

### (3) Production of gene recombinant antibody

One preferable aspect of the present invention comprises a gene recombinant antibody. Examples of the gene recombinant antibody include, but not limited to, a chimeric antibody, a human-type antibody and a humanized antibody.

A chimeric antibody (namely, a human-type chimeric antibody) is an antibody in which a variable region of a mouse-derived antibody is linked (joined) to a constant region of a human-derived antibody (see Proc. Natl. Acad. Sci. U.S.A. 81, 6851-6855, (1984), etc.), where a chimera can be produced easily by constructing an antibody linked as above by a gene recombinant technique.

If a human-type antibody is to be prepared, a so-called CDR grafting technique can be employed. A process of producing such a human-type antibody is well known in the art (see Nature, 321, 522-525 (1986); J. Mol. Biol., 196, 901-917 (1987); Queen C et al., Proc. Natl. Acad. Sci. USA, 86: 10029-10033 (1989); Japanese Patent Publication No. 2828340, etc.).

In general, a human antibody (complete human antibody) refers to one that has the same structures as the hypervariable region (an antigen-binding site of the variable region (V region)), other parts of the V region and the constant region of a human antibody. Techniques for producing a human antibody are also known, and a method for producing a gene sequence common in human has been established through genetic engineering. A human antibody may be acquired, for example, by a method that employs a human antibody-producing mouse having a human chromosome fragment containing genes of heavy chain (H chain) and light chain (L chain) of a human antibody (see Tomizuka, K. et al., Nature Genetics, (1977)16, 133-143; Kuroiwa, Y. et al., Nuc. Acids Res., (1998)26, 3447-3448; Yoshida, H. et al., Animal Cell Technology: Basic and Applied Aspects, (1999)10, 69-73 (Kitagawa, Y., Matuda, T. and Iijima, S. eds.), Kluwer Academic Publishers; Tomizuka, K. et al., Proc. Natl. Acad. Sci. USA, (2000)97, 722-727, etc.), or by a method in which a human antibody is acquired by screening human antibody-displaying phage libraries (see Wormstone, I. M. et al, Investigative Ophthalmology & Visual Science., (2002)43 (7), 2301-8; Carmen, S. et al., Briefings in Functional Genomics and Proteomics, (2002)1 (2), 189-203; Siriwardena, D. et al., Opthalmology, (2002)109 (3), 427-431, etc.).

### (4) Production of antibody fragment

Examples of an antibody fragment against an influenza virus-derived protein used for the present invention include Fab, Fab', F(ab')2, Fv, diabody (dibodies), dsFv and scFv (single chain Fv). These antibody fragments can be obtained by cleaving the antibody of the present invention with a variety of proteases in accordance with the purpose.

For example, Fab can be obtained by treating the antibody molecule with papain while F(ab')2 can be obtained by treating the antibody molecule with pepsin. Fab' can be obtained by cleaving the disulfide bond at the hinge region of F(ab')2 mentioned above. In the case of scFv, cDNA coding for heavy chain variable region (H chain V region) and light chain variable region (L chain V region) of the antibody is acquired to construct DNA coding for scFv. This DNA is inserted into an expression vector, and the resulting expression vector is introduced into and expressed in a host animal to produce scFv.

In the case of a diabody, cDNA coding for H chain V region and L chain V region of the antibody is acquired to construct DNA coding for scFv such that the length of the amino acid sequence of the peptide linker is 8 residues or less. This DNA is inserted into an expression vector, and the resulting expression vector is introduced into and expressed in a host animal to produce a diabody. In the case of dsFv, cDNA coding for H chain V region and L chain V region of the antibody is acquired to construct DNA coding for dsFv. This DNA is inserted into an expression vector, and the resulting expression vector is introduced into and expressed in a host animal to produce dsFv.

### 5. H5N1 HPAIV vaccine (rDIs-H5 HA) or inactivated vaccine

A method for producing a H5N1 HPAIV vaccine is known (Japanese Patent No. 5884100), and this known method can be employed for the production. The outline of the production method will be described below.

The HA genes of H5N1 HPAIV have already been submitted and assigned with accession numbers (Accession Nos.) in the GenBank database (http://www.ncbi.nlm.nih.gov/genbank/) provided by the National Center for Biotechnology Information (NCBI).

For example, as shown in Table 1 below, genes coding for HA proteins derived from respective H5N1 HPAIV clades, and genes having the DNA coding for the HA proteins of respective H5N1 HPAIV clades partially knocked out are assigned with the GenBank accession numbers.

**[Table 1]**

| | Accession number |
|---|---|
| Subtype H5, clade 1 (mC11) | EF541402 |
| Subtype H5, clade 2.1 (mC12.1) | EF541394 |
| Subtype H5, clade 2.2 (mC12.2) | |
| Subtype H5, clade 2.3 (mC12.3) | DQ371928 |
| Subtype H1, H1N1(2009) pdm | FJ969540 |
| mCl1, partially knocked out | - |
| mCl 2.1, partially knocked out | - |
| mCl 2.2, partially knocked out | DQ659327 |
| mCl 2.3, partially knocked out | - |

These HA genes (including the partially knocked out and mutated genes) have already been cloned and inserted into plasmids. Therefore, the whole (including the mutated sequences) or a part of the gene contained in the recombinant vaccinia virus of the present invention can be obtained by general genetic engineering.

The above method may be site-directed mutagenesis described in "Molecular Cloning, A Laboratory Manual (4th edition)" (Cold Spring Harbor Laboratory Press (2012)) or "Current Protocols in Molecular Biology, John Wiley & Sons (1987-1997)" or the like, or the gene may be prepared using a mutagenesis kit utilizing Kunkel method, Gapped duplex method or the like.

The expression promoter contained in the recombinant vaccinia virus of the present invention is not limited as long as it can be inserted into a gene knocked out region of the vaccinia virus DIs strain, and it may, for example, be vaccinia virus promoter mH5 (Virology. vol. 351, p. 368-380). As said expression promoter, for example, a hybrid promoter consisting of a poxvirus A-type inclusion (ATI) promoter and repeats of a vaccinia virus 7.5 kDa protein (p7.5) early expression promoter may be used. This promoter can be linked to a suitable plasmid. For example, pUC/DIs is known (see Arch. Virol. vol. 138, p. 315-330, 1994; Japanese Unexamined Patent Application Publication No. Heisei 6-237773).

A plasmid vector obtained by inserting DNA coding for the expression promoter and the HA protein is introduced into a vaccinia virus to produce a recombinant vaccinia virus. The plasmid vector can be introduced into the host by employing any known technique. For example, the resulting plasmid vector can be introduced into a chicken fibroblast infected with an attenuated vaccinia virus DIs strain to produce a recombinant vaccinia virus (rDIs/mCl1, rDIs/mC12.1, rDIs/mC12.2 and rDIs/mC12.3).

Methods for producing an inactivated vaccine are known. As mentioned above, an influenza virus used for the vaccine production is propagated by inoculating embryonated chicken eggs with the virus, then the virus purified/concentrated from the chorioallantoic fluid is partially degraded with ether, and the resultant is further inactivated with formalin.

### 6. Medication for preventing and treating new type of influenza and mechanism of infection protection

The present invention provides a medication for preventing or treating influenza, which comprises: (i) the above-described recombinant vaccinia virus derived from a DIs strain or inactivated virus; or (ii) a combination of said recombinant vaccinia virus or inactivated virus and an antibody against an influenza virus-derived protein.

The present invention further provides a pharmaceutical composition for preventing or treating influenza, which comprises at least one component selected from the group consisting of (a)-(d) below:
(a) an activated CD4/CD8-positive cell
(b) an activator of a CD4/CD8-positive cell
(c) a combination of a CD4-positive cell and an activator of a CD4-positive cell
(d) a combination of a CD8-positive cell and an activator of a CD8-positive cell

In addition to any one or a combination of (a)-(d) above, the medication of the present invention may further comprise (e) an antibody against an influenza virus-derived protein, (f) the above-described recombinant vaccinia virus derived from a DIs strain or inactivated virus, or (g) a combination of said recombinant vaccinia virus or inactivated virus and said antibody.

The present invention further provides: a method for preventing or treating influenza, comprising a step of administering the medication to a patient (subject); use of the medication for preventing and treating influenza; use of the medication for manufacturing a medicine for preventing and treating a new type of influenza.

The pharmaceutical composition of the present invention may be introduced into a living body by a known method such as an intramuscular, intraperitoneal, intradermal or subcutaneous injection, a nasal, oral or pulmonary inhalation, or an oral administration.

Moreover, the antibody, the CD4/CD8-positive cell, the activator of the CD4/CD8-positive cell or a combination thereof contained in the pharmaceutical composition of the present invention may be used in combination with the above-described recombinant vaccinia virus or inactivated virus.

The aspect of such combinational use is not particularly limited, and the medication of the present invention can be administered simultaneously with the recombinant vaccinia virus or the inactivated virus of the present invention, or they can be introduced into a living body by administering one after the other after a certain lapse of time.

According to the present invention, if the antibody, the CD4/CD8-positive cell, the activator of the CD4/CD8-positive cell or a combination thereof is to be used as a pharmaceutical composition, it may be mixed with a known pharmaceutically acceptable carrier such as an excipient, a filler, a binder or a lubricant, a buffer, a tonicity agent, a chelating agent, a coloring agent, a preservative, a fragrance, a flavoring agent, a sweetener or the like.

The pharmaceutical composition of the present invention can be administered orally or parenterally according to the dosage form, for example, as an oral administration agent such as tablets, capsules, powder, granules, pills, a liquid agent or a syrup, or as a parenteral administration agent such as an injection, a topical medication, a suppository or eye drops. Preferable examples include local injections such as intradermal, intramuscular and intraperitoneal injections.

The dosage may appropriately be selected according to the type of the active element, the administration route, the administration target, the age, weight, sex and symptoms of the patient, and other conditions.
Antibody: Intravenous
Activated CD4/CD8-positive cell: Intravenous
Activator of CD4/CD8-positive cells: Oral, intravenous

The daily dosage of the virus is about 1000-1000000000 PFU (plaque forming units) and preferably about 100000-100000000 PFU for oral administration, and about 100-1000000000 PFU and preferably about 1000-100000000 PFU for parenteral administration. The virus may be given once or in several times a day.

The recombinant vaccinia virus or the inactivated virus of the present invention can be used as a vaccine for preventing and treating influenza. Conventionally, the neutralizing antibody titer against H5N1 HPAIV has been considered important for the development of a vaccine against H5N1 HPAIV. On the other hand, it was found that the recombinant vaccinia virus of the present invention can exert an effective protective effect even under a circumstance where the neutralizing antibody titer is hardly induced, and that cell groups and factors involved in the infection protection vary with days following the H5N1 HPAIV infection.

Specifically, in an individual immunized with the H5N1 HPAIV vaccine (rDIs/mC12.2), the virus is generally eliminated to the detection limit or less by 6 days after the H5N1 HPAIV infection. In this vaccinated individual, the binding antibody and a cell group having a Fc gamma receptor contribute to the protection against virus infection until a day after the H5N1 HPAIV infection.

On the other hand, as can be appreciated from the results of the CD4-positive cell depletion experiment, the CD4-positive cell was found to be essential for the protection against virus infection from Day 1 to 3 following the infection. Furthermore, CD8-positive cells were found to be necessary for the protection against virus infection from Day 3 to 6 following the infection.

Accordingly, the recombinant vaccinia viruses prepared by the present inventors (collectively referred to as "Flu HA-RVVs") can exert a protective effect against H5N1 HPAIV infection by an action mechanism that differs from that of an antigen-specific antibody (especially, a neutralizing antibody) which has been considered important for the preventive action of the conventional inactivated whole particle vaccination.

### 7. Kit

A kit of the present invention can comprise a RVV (rDIs) or an inactivated virus, an antibody against an influenza virus-derived protein, or a combination thereof.

In addition, the kit of the present invention comprises any or a combination of (a)-(d) below:
(a) an activated CD4/CD8-positive cell
(b) an activator of a CD4/CD8-positive cell
(c) a combination of a CD4-positive cell and an activator of a CD4-positive cell
(d) a combination of a CD8-positive cell and an activator of a CD8-positive cell

In addition to (a)-(d) above, the kit of the present invention may further comprise (e) an antibody against an influenza virus-derived protein, (f) a RVV (rDIs) or an inactivated virus, or (g) a combination of said recombinant vaccinia virus or inactivated virus and said antibody.

The kit of the present invention can be used for preventing or treating highly pathogenic H5N1 avian influenza.

Hereinafter, the present invention will be described more specifically by way of examples. These examples are provided merely for illustration and are not intended to limit the scope of the present invention.

### EXAMPLE 1

### <Method>

### 1) Production of DIs strain-derived recombinant vaccinia virus that has been introduced with influenza virus hemagglutinin gene

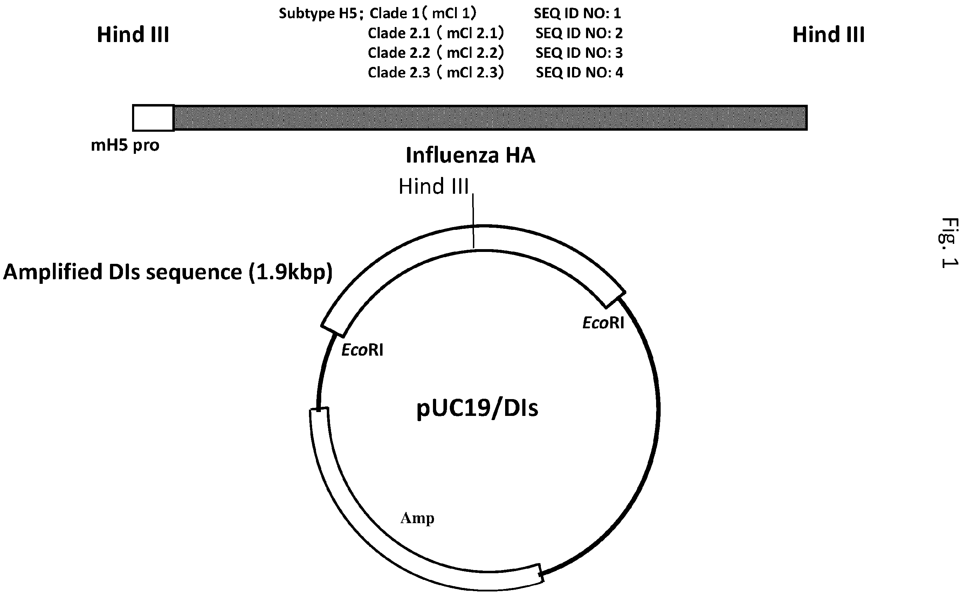

A hemagglutinin protein (HA) gene, namely, an antigen of an artificially synthesized H5N1 HPAIV, was linked downstream from a vaccinia virus promoter mH5 sequence, and the resultant was inserted into a plasmid vector for homologous recombination (Figure 1). For insertion of the subtype H5N1 HA sequence, Hind III site was used as the restriction enzyme site. The plasmid vector produced for homologous recombination (linearized by cleaving a single site with a restriction enzyme) was transfected into CEF that had been infected with a DIs strain in advance to cause homologous recombination in the region abutting the gene knocked out site of the DIs strain to produce a recombinant vaccinia virus (RVV) in which the mH5 promoter and the influenza virus HA gene were inserted into the genome of the vaccinia virus, specifically, various RVVs, namely, rDIs/mCl1, rDIs/mC12.1, rDIs/mC12.2 and rDIs/mCl2.3. While the influenza virus-derived HA protein is involved in agglutination reaction of guinea pig red blood cells, the vaccinia virus-derived HA protein does not cause agglutination reaction of guinea pig red blood cells. Therefore, RVV was screened by infecting CEF with the above-described recombinant virus and using agglutination reaction of guinea pig red blood cells to the resulting plaque as an indicator. Those that formed red plaques having hemagglutination activity were selected as the recombinant virus of interest.

### 2) Protective effect against H5N1 HPAIV infection in mice given single immunization with DIs strain-derived recombinant vaccinia virus

Mice were intradermally immunized with 1 x 10⁷ PFU of the recombinant vaccinia virus (rDIs/mC12.2) on the back. H5N1 HPAIV challenge infection was conducted 4 weeks after the immunization, and the weight and the survival rate were determined daily. Furthermore, blood and lung tissue samples were collected on Day 1, 3, 6 and 9 to determine the viral loads in the lung tissues.

### 3) Determination of neutralizing antibody titer

The collected sera were used to determine the neutralizing titers in the sera by using the same virus strain as that of H5N1 HPAIV used for infecting the mice.

### 4) H5N1 HPAIV infection of mice transferred with anti-H5N1 HPAIV binding antibody

Following H5N1 HPAIV challenge infection of mice transferred with anti-H5N1 HPAIV binding antibody, lung tissue samples were collected on Day 1 and 6 after the infection to determine the viral titers in the lung tissues.

### 5) H5N1 HPAIV challenge infection of wild-type mice and FcR gamma knockout mice given single vaccination

FcR gamma knockout mice and wild-type mice were intradermally immunized with 1 x 10⁷ PFU of the recombinant vaccinia virus (rDIs/mC12.2) on the back. H5N1 HPAIV challenge infection was conducted 4 weeks after the immunization, and the weight and the survival rate were determined daily. Furthermore, lung tissue samples were collected on Day 1 and 3 to determine the viral titers in the lung tissues.

### 6) Role of CD4-positive cell in protection effect against infection by single immunization with DIs strain-derived recombinant vaccinia virus

BALB/c mice were intradermally immunized with 1 x 10⁷ PFU of the recombinant vaccinia virus (rDIs/mC12.2) on the back. H5N1 HPAIV challenge infection was conducted 4 weeks after the immunization. In advance of the challenge infection, a CD4-positive cell depleting antibody was intravenously administered every other day starting 2 days ahead of the infection. The weight and the survival rate were determined daily. Furthermore, lung tissue samples were collected on Day 1, 3 and 6 to determine the viral loads in the lung tissues.

### 7) Role of CD8-positive cell, or CD4-positive cell and CD8-positive cell in protection effect against infection by single immunization with DIs strain-derived recombinant vaccinia virus

BALB/c mice were intradermally immunized with 1 x 10⁷ PFU of the recombinant vaccinia virus (rDIs/mCl2.2) on the back. H5N1 HPAIV challenge infection was conducted 4 weeks after the immunization. In advance of the challenge infection, a CD8-positive cell depleting antibody, or both CD4- and CD8-positive cell depleting antibodies were intravenously administered every other day starting 2 days ahead of the infection. Lung tissue samples were collected on Day 1, 3 and 6 to determine the viral loads in the lung tissues.

### 8) Influence of depletion of both CD4- and CD8-positive cells in single immunization with DIs strain-derived recombinant vaccinia virus for acquiring protective effect against infection on weight and survival rate

BALB/c mice were intradermally immunized with 1 x 10⁷ PFU of the recombinant vaccinia virus (rDIs/mC12.2) on the back. H5N1 HPAIV challenge infection was conducted 4 weeks after the immunization. In advance of the challenge infection, both CD4- and CD8-positive cell depleting antibodies were intravenously administered every other day starting 2 days ahead of the infection. The weight and the survival rate were determined daily.

### <Results>

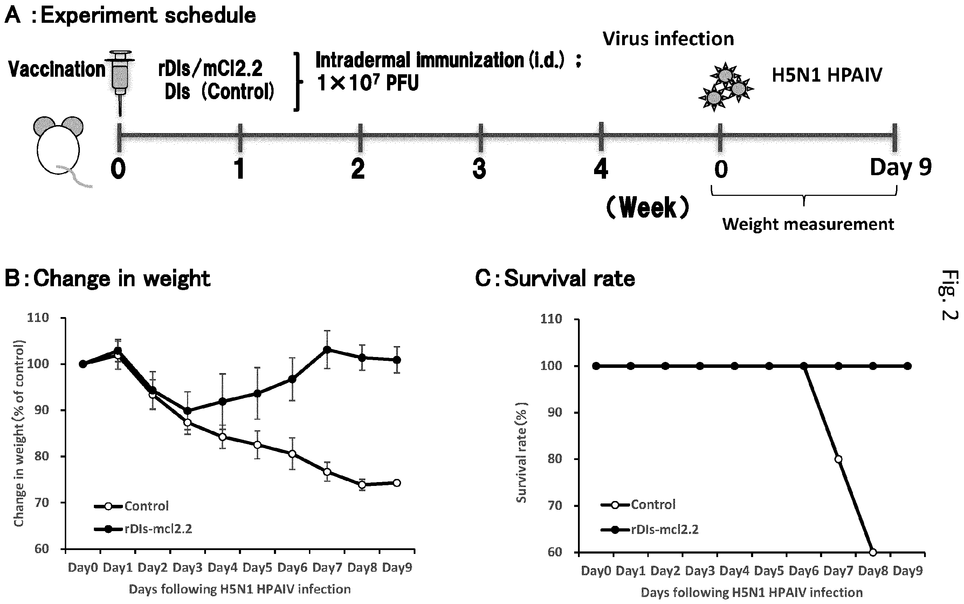

Although temporal weight loss was observed after the challenge infection with H5N1 HPAIV in the mice that had been given a single immunization with the prepared RVV (rDIs/mC12.2), they rapidly gained weight and showed 100% survival rate. On the other hand, for the control group that did not receive the vaccination, rapid weight loss was observed and all of the mice died (Figure 2). At this point, the viral titers in the lung tissues of the control group and the vaccinated group were determined. As a result, the viral titer was high until 9 days after the infection for the control group, whereas the viral titer was reduced to the detection limit or less in most of the individuals by 6 days after the infection for the vaccinated group (Figure 3).

Meanwhile, similar to the control group, the neutralizing antibody titer against the virus strain used for the challenge infection was not detected in the vaccinated individuals in which the virus was rapidly eliminated in the lung tissues (Figure 4).

Thus, for the purpose of specifically analyzing the mechanism of the protective action in the vaccinated individuals, IgG contained in the antisera collected from the vaccinated individuals was purified and intravenously administered to naive mice, which were then subj ected to H5N1 HPAIV challenge infection. As a result, similar to the H5N1 HPAIV infection of the vaccinated individuals, the viral titers in the lung tissues decreased to 1/5 to 1/10 of that of the non-vaccinated individuals on Day 1 following the infection (Figure 5). Next, for the purpose of evaluating whether or not the cell group having FcR that binds to antigen-specific IgG was involved in this viral titer reduction at the early stage of infection, FcR gamma knockout mice were vaccinated and subjected to H5N1 HPAIV challenge infection.

When the viral titers in the lung tissues on Day 1 following the infection were compared to those of the wild-type mice, the viral titers in the lung tissues of the FcR gamma knockout mice were higher than those of the wild-type mice (Figure 6). From the results of the experiment employing antigen-specific IgG transfer and the experiment using FcR gamma knockout mice, the reduction of the viral titers in the lung tissues of the vaccinated individuals on Day 1 following the infection was found to be the effect exerted by the cell group having the binding antibody and FcR. Moreover, the CD4-positive cell group was depleted by intravenously administering an anti-CD 4 antibody to the vaccinated individuals every other day during the infection phase starting 2 days ahead of the H5N1 HPAIV challenge infection.

For the CD4-positive cell depleted group, the viral titers in the lung tissues on Day 3 following the H5N1 HPAIV infection were high. From this result, the CD4-positive cell was found to play an important role in the virus elimination from Day 1 to 3 following the infection. Moreover, infectious virus was detected even 6 days after the infection in the group depleted of the CD4-positive cell but the titers thereof were lower than those of the non-vaccinated individuals. Therefore, it was found that, in addition to the CD4-positive cell group, other cell group or factor was necessary for the virus elimination from Day 3 to 6 following the infection (Figure 7).

Next, the CD8-positive cell or both of the CD4- and CD8-positive cells were depleted from the vaccinated individuals. As a result, virus was not eliminated 6 days after the infection in both of the CD8 cell-depleted group and the CD4/CD8 cell-depleted groups (Figure 8). Furthermore, the individuals of the CD4/CD8 cell-depleted group gradually lost weight and some died even though they had been vaccinated (Figure 9).

### <Discussion>

As a H5N1 HPAIV vaccine (rDIs/mC12.2), a single immunization with 1 x 10⁷ PFU of the DIs strain-derived RVV expressing influenza virus HA gene showed rapid weight gaining and 100% survival rate against lethal H5N1 HPAIV challenge infection. Hence, the DIs strain-derived RVV also has sufficient effectiveness as a H5N1 HPAIV vaccine. In individuals immunized with the H5N1 HPAIV vaccine (rDIs/mC12.2), virus was generally eliminated to the detection limit or less by 6 days after the H5N1 HPAIV infection. In these vaccinated individuals, the cell group having the binding antibody and the Fc gamma receptor was found to play an important role in protection against the virus infection until a day after the H5N1 HPAIV infection. On the other hand, the CD4-positive cell was found to be essential for the protection against the virus infection from Day 1 to 3 following the infection from the results of the CD4-positive cell depletion experiment.

Moreover, for the protection against the virus infection from Day 3 to 6 following the infection, the CD4- and CD8-positive cells possibly contributed to the virus elimination. Some of the individuals depleted of the CD4/CD8-positive cells died even though they were vaccinated. These results showed that the stepwise action of the CD4-and CD8-positive cells, i.e., an action mechanism different from the neutralizing antibody which has been considered important in the preventive action of a conventional inactivated whole particle vaccination, exerted the protective effect against the H5N1 HPAIV infection.

From the above-described results, the DIs strain-derived RVV expressing an influenza virus HA gene of the present invention was found to serve as a safe and effective vaccine against rapidly spreading influenza virus. Furthermore, the CD4- and the CD8-positive cells were found to function stepwisely as the protection mechanism of said vaccine.

## Claims

1. A medication for preventing or treating influenza, comprising a recombinant vaccinia virus that contains an expression promoter and the entire or a part of DNA coding for an influenza virus-derived protein within a genome of a vaccinia virus DIs strain, or an inactivated influenza virus.

2. The medication according to Claim 1, wherein influenza is prevented or treated by the action of an activated CD4-positive cell and/or an activated CD8-positive cell.

3. The medication according to either one of Claims 1 and 2, further comprising an antibody against an influenza virus-derived protein.

4. The medication according to Claim 3, wherein the antibody is a binding antibody.

5. The medication according to any one of Claims 1-4, wherein the influenza virus-derived protein is a hemagglutinin protein derived from highly pathogenic H5N1 avian influenza virus.

6. A medication for preventing or treating influenza, comprising any one of components (a)-(c) below:
(a) at least one component selected from the group consisting of an activated CD4-positive cell, an activator of a CD4-positive cell and a combination of a CD4-positive cell and an activator of a CD4-positive cell;
(b) at least one component selected from the group consisting of an activated CD8-positive cell, an activator of a CD8-positive cell and a combination of a CD8-positive cell and an activator of a CD8-positive cell; and
(c) a component consisting of a combination of the components (a) and (b).

7. The medication according to Claim 6, further comprising a recombinant vaccinia virus that contains an expression promoter and the entire or a part of DNA coding for an influenza virus-derived protein within a genome of a vaccinia virus DIs strain, or an inactivated influenza virus.

8. The medication according to either one of Claims 6 and 7, further comprising an antibody against an influenza virus-derived protein.

9. The medication according to Claim 8, wherein the antibody is a binding antibody.

10. The medication according to any one of Claims 7-9, wherein the influenza virus-derived protein is a hemagglutinin protein derived from highly pathogenic H5N1 avian influenza virus.

11. A kit for preventing or treating influenza, comprising a recombinant vaccinia virus that contains an expression promoter and the entire or a part of DNA coding for an influenza virus-derived protein within a genome of a vaccinia virus DIs strain, or an inactivated influenza virus.

12. The kit according to Claim 11, wherein influenza is prevented or treated by the action of an activated CD4-positive cell and/or an activated CD8-positive cell.

13. The kit according to either one of Claims 11 and 12, further comprising an antibody against an influenza virus-derived protein.

14. The kit according to Claim 13, wherein the antibody is a binding antibody.

15. The kit according to any one of Claims 11-14, wherein the influenza virus-derived protein is a hemagglutinin protein derived from highly pathogenic H5N1 avian influenza virus.

16. A kit for preventing or treating influenza, comprising any one of components (a)-(c) below:
(a) at least one component selected from the group consisting of an activated CD4-positive cell, an activator of a CD4-positive cell and a combination of a CD4-positive cell and an activator of a CD4-positive cell;
(b) at least one component selected from the group consisting of an activated CD8-positive cell, an activator of a CD8-positive cell and a combination of a CD8-positive cell and an activator of a CD8-positive cell; and
(c) a component consisting of a combination of the components (a) and (b).

17. The kit according to Claim 16, further comprising a recombinant vaccinia virus that contains an expression promoter and the entire or a part of DNA coding for an influenza virus-derived protein within a genome of a vaccinia virus DIs strain, or an inactivated influenza virus.

18. The kit according to either one of Claims 16 and 17, further comprising an antibody against an influenza virus-derived protein.

19. The kit according to Claim 18, wherein the antibody is a binding antibody.

20. The kit according to any one of Claim 17-19, wherein the influenza virus-derived protein is a hemagglutinin protein derived from highly pathogenic H5N1 avian influenza virus.
